(19) Europäisches Patentamt
European Patent Office
Office européen des brevets

(11) **EP 4 275 657 A1**

(12) # EUROPEAN PATENT APPLICATION

(43) Date of publication:
**15.11.2023 Bulletin 2023/46**

(21) Application number: **23173220.7**

(22) Date of filing: **12.05.2023**

(51) International Patent Classification (IPC):
**A61F 2/30** (2006.01)   **B33Y 80/00** (2015.01)

(52) Cooperative Patent Classification (CPC):
**A61F 2/30771; A61F 2/3094; B33Y 80/00;**
A61F 2002/30011; A61F 2002/3092;
A61F 2002/30968; A61F 2002/3097;
A61F 2002/30971; A61F 2002/30985

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **12.05.2022 US 202263341092 P**

(71) Applicant: **Howmedica Osteonics Corporation**
**Mahwah, New Jersey 07430 (US)**

(72) Inventors:
• **DELUISE, Michael A.**
  **Paramus, 07652 (US)**
• **HEARD, David William**
  **Summit, 07901 (US)**
• **PATEL, Soniya**
  **Springfield, 07081 (US)**
• **HAGGERTY, Zz Mae**
  **Montclair, 07042 (US)**

(74) Representative: **Gill Jennings & Every LLP**
**The Broadgate Tower**
**20 Primrose Street**
**London EC2A 2ES (GB)**

(54) **FATIGUE RESISTANT POROUS STRUCTURE**

(57)    At least a portion of an object such as a medical implant is fabricated by a process. In the process, a porous structure, a solid structure, and an interface region directly attached to each of the porous structure and the solid structure are produced by an additive manufacturing machine using a stored output file configured for providing instructions to the additive manufacturing machine for fabricating the porous structure, the solid structure, and the interface region. The stored output file is prepared by preparing a computer-generated component file including a porous CAD volume (114) and a solid CAD volume. Digitized radii (119, 219, 319) are added to digitized struts defining digitized pores in an interface volume of porous CAD volume to mitigate stress concentrations that would otherwise result in sharp corners or notches in the fabricated object.

FIG. 4C

EP 4 275 657 A1

## Description

CROSS-REFERENCE TO RELATED APPLICATION

The present application claims the benefit of the filing date of U.S. Provisional Patent Application No. 63/341,092 filed May 12, 2022, the disclosure of which is hereby incorporated herein by reference.

BACKGROUND

Additive manufacturing (AM) is currently driving significant change within the medical device industry. AM processing allows for the design and production of a component with complex geometries not achievable by conventional (casting/machining) production methods, such as titanium lattice or thicket porous ingrowth surfaces which have demonstrated suitable biological fixation by bone ingrowth.

However, these complex ingrowth structures can create surface geometries similar to that of a geometric notch. These localized notches occurring at the junction of porous surfaces and generally solid substrates may result in the formation of localized stress concentration points along a component having such notches. In the presence of a notch, the maximum stress ($\sigma_{max}$) realized within the component can be estimated by the following equation, wherein Kt is the theoretical stress concentration factor of the notch and is determined based on the geometry of the notch, and wherein $\sigma_{nom}$ is the nominal stress according to the following:

$$\sigma_{max} = K_t \times \sigma_{nom} \text{ (“Equation 1”)}.$$

Further complicating the issue is the notch sensitivity of certain biocompatible materials that have otherwise desirable properties for medical implants, such as certain titanium alloys. When AM or other complex ingrowth structures are combined with the notch sensitivity of certain implant materials, a reduction in component fatigue strength on the order of 50% can occur. This component level fatigue strength reduction has limited the potential number of clinical applications of complex ingrowth structures and therefore limits both the potential benefit to patients and the proliferation of a novel technology.

Accordingly, improvements in fatigue strength for components having porous-solid interfaces are needed.

BRIEF SUMMARY

According to some aspects, a computer model of a design for a three-dimensional porous-solid object, e.g., a medical implant, may include a digitized solid body or substrate portion and a digitized osteointegrative portion including digitized porous geometries on at least one surface of the solid body or substrate portion. The digitized osteointegrative portion may be modified via modification of the porous geometries with digitized fillets. Likely locations for potential notches within the object may be identified by determining stress concentrations within the computer model for the object, e.g., using finite element analysis, especially such stress concentrations at an interface of a solid body or substrate portion corresponding to the digitized solid body or substrate portion and and a osteointegrative portion corresponding to the digitized osteointegrative portion. The locations where notches are likely to occur may also be identified by algorithmically finding geometric features meeting certain criteria. In some arrangements, the fillets may be placed only within an interface portion extending between the osteointegrative portion and the solid body or substrate portion. The interface portion may be entirely contiguous with the solid body or substrate portion. In some arrangements, radius values of the digitized fillets within the interface portion may be uniform. In some other arrangements, radius values of the digitized fillets may vary and be calculated in each case to bring local stress concentration factors within acceptable limits.

In another aspect, a three-dimensional poroussolid object, e.g., medical implant, may comprise a porous structure including a plurality of struts defining pores. The object may also comprise a solid structure. The object may also comprise an interface region attaching the porous structure to the solid structure. The interface region may include a plurality of radii. Each of the radii may be directly attached to a strut of the plurality of struts of the porous structure and directly attached to the solid structure such that the porous structure and the solid structure are attached only by the plurality of radii.

In another aspect, a three-dimensional poroussolid object, e.g., a medical implant, may include a porous structure and a solid structure. The porous structure may include a plurality of struts that may define pores. The object may also include an interface region that may attach the porous structure to the solid structure. The interface region may include a plurality of radii. Each of the radii may be directly attached only to a respective strut of the plurality of struts of the porous structure and to the solid structure such that the porous structure and the solid structure may be directly attached only to each other and by the plurality of radii.

In some arrangements according to any of the foregoing aspects, the medical implant may be in the form of a shoulder implant, a hip stem component, a patellofemoral component, a tibial component, a spinal implant, a knee implant, a trauma plate, a foot implant, an ankle implant, or an acetabular cup.

In some arrangements according to any of the foregoing, the porous structure, the solid structure, and the plurality of radii all may be made of the same material.

In some arrangements according to any of the foregoing, the plurality of radii may be defined by the same radius value.

In some arrangements according to any of the foregoing, radius values of the plurality of radii may vary.

In some arrangements according to any of the foregoing, the plurality of radii may include adjacent radii directly attached to adjacent struts of the plurality of struts, the adjacent radii being directly attached to each other.

In some arrangements according to any of the foregoing, the adjacent radii may be defined by the same radius value.

In some arrangements according to any of the foregoing, radius values of the adjacent radii vary.

In some arrangements according to any of the foregoing, all of the radii may be defined by the same radius value.

In some arrangements according to any of the foregoing, the porous structure, the solid structure, and the interface region may form an integral structure such that the porous structure, the solid structure, and the interface region are inseparable without fracture of any one or any combination of the porous structure, the solid structure, and the interface region.

In some arrangements according to any of the foregoing, the object may be prepared using a stored output file configured for providing instructions to an additive manufacturing machine for fabricating the object, the porous structure, the solid structure, and the interface region forming at least part of the object. The output file may be prepared by a process. In such process, a plurality of digitized struts corresponding to formed struts of the porous structure of the object and defining a porous CAD volume may be formed by one or more computer processors. A solid model region corresponding to the solid structure of the object and defining a solid CAD volume may be formed by the one or more computer processors. Digitized radii corresponding to physical radii of the interface region of the object to be formed and defining an interface volume, the digitized radii being directly attached to the plurality of digitized struts and to the solid model region, may be formed by the one or more computer processors. A computer-generated model of the object configured for additive manufacturing, the computer-generated model including data corresponding to the digitized struts, the solid model region, and the digitized radii, may be generated by the one or more computer processors. The computer-generated model of the object may be stored into the output file by the one or more computer processors.

In some arrangements according to any of the foregoing, any one or any combination of the radii may have a radius value approximately equal to 0.025 mm, 0.05 mm, 0.075 mm, 0.10 mm, 0.25 mm, or 0.50 mm.

In another arrangement according to any of the foregoing, any one or any combination of the radii may have a radius value of 0.50 mm or less.

In another arrangement according to any of the foregoing, any or all of the radii may have a radius value in a range from 0.025 mm to 0.05 mm.

In another arrangement according to any of the foregoing, the interface region may attach the porous structure to a surface of the solid structure, and portions of the surface extending between at least some of the radii may not be contacted by any of the struts or by any of the radii.

In another aspect, a method of fabrication of a three-dimensional porous-solid object, e.g., medical implant, may comprise producing a porous structure, a solid structure, and an interface region directly attached to each of the porous structure and the solid structure using a stored output file configured for providing instructions to an additive manufacturing machine for fabricating the object, the porous structure, the solid structure, and the interface region forming at least part of the object. The output file may be prepared by a process. In such process, a plurality of digitized struts corresponding to formed struts of the porous structure of the object and defining a porous CAD volume may be formed by one or more computer processors. A solid model region corresponding to the solid structure of the object and defining a solid CAD volume may be formed by the one or more computer processors. Digitized radii corresponding to physical radii of the interface region of the object to be formed and defining an interface volume, the digitized radii being directly attached to the plurality of digitized struts and to the solid model region may be formed by the one or more computer processors. A computer-generated model of the object configured for additive manufacturing, the computer-generated model including data corresponding to the digitized struts, the solid model region, and the digitized radii, may be generated by the one or more computer processors. The computer-generated model of the object may be stored into the output file by the one or more computer processors.

In some arrangements according to the foregoing aspect, the output file may be further prepared by the step of identifying concave areas at which digitized struts meet the solid model region.

In some arrangements according to any of the foregoing, the entirety of the interface volume may be contiguous with the solid CAD volume.

In some arrangements according to any of the foregoing, the interface volume may be defined within a predetermined distance from the solid CAD volume.

In some arrangements according to any of the foregoing, the interface volume may have a thickness that is of a predetermined proportion to a thickness of the porous CAD volume as measured normal to a surface of the solid CAD volume with which the interface volume is contiguous across an area of the surface of the solid CAD volume.

In some arrangements according to any of the foregoing, the identifying of the concave notches may include conducting finite element analysis of at least part of the porous CAD volume to find stress concentrations having characteristics consistent with concave notches in the fabricated object.

In some arrangements according to any of the foregoing, the forming of the digitized radii may include setting all of the digitized radii to a same radius setting corresponding to a radius value for the physical radii of the interface region of the object.

In some arrangements according to any of the foregoing, the forming of the digitized radii may include placing, by the one or more computer processors, each of the digitized radii at respective locations within the interface volume having a determined initial stress concentration factor above a preset minimum threshold and assigning, by the one or more computer processors, each of the respective digitized radii a respective radius setting that reduces the determined initial stress concentration factor at the respective locations to a determined new stress concentration factor that may be less than or equal to a preset maximum threshold.

In some arrangements according to any of the foregoing, the preset minimum threshold may equal the preset maximum threshold. In some such arrangements, each of the digitized radii at respective locations within the interface volume having a determined initial stress concentration factor above the preset minimum threshold may be assigned a respective radius setting that reduces the determined new stress concentration factor to less than the preset maximum threshold.

In some arrangements according to any of the foregoing, the forming of the digitized radii may include setting radii settings for the digitized radii based on multiple factors. At least two of the multiple factors may be selected from any one or any combination of respective angles between the digitized struts onto which the digitized radii are being formed and the solid model region, the respective thicknesses of the digitized struts, the material of the porous structure of the object to be fabricated, the material of the solid structure of the object to be fabricated, and the material of the physical radii to be fabricated.

In some arrangements according to any of the foregoing, any one or any combination of the digitized radii may be set to a radius setting corresponding to a radius value for the physical radii of the interface region of the object of approximately equal to 0.025 mm, 0.05 mm, 0.075 mm, 0.10 mm, 0.25 mm, or 0.50 mm.

In some arrangements according to any of the foregoing, any one or any combination of the digitized radii may be set to a radius setting corresponding to a radius value for the physical radii of the interface region of the object of 0.5 mm or less.

In some arrangements according to any of the foregoing, any one or any combination of the digitized radii may be set to a radius setting corresponding to a radius value for the physical radii of the interface region of the object in a range from 0.025 mm to 0.05 mm.

In some arrangements according to any of the foregoing, the forming of the digitized radii may leave portions of a digitized surface of the solid model region extending between at least some of the digitized radii free of contact with any of the digitized struts and with any of the digitized radii in which the digitized surface of the solid model region corresponds to a surface of the solid structure. In this manner, the forming of the digitized radii may leave portions of the surface of the solid structure extending between the respective digitized radii free of contact with any of the struts and with any of the radii when the object is formed.

In another aspect, a three-dimensional object, e.g., a medical implant, may be designed by a process. In this process, a digitized substrate corresponding to a body portion of the object may be created via one or more computer processors. In the process, a digitized osteointegrative volume populated with digitized porous geometries corresponding to porous geometries of the object and corresponding to an osteointegrative portion of the object may be created via the one or more computer processors. The digitized osteointegrative volume may be contiguous with the digitized substrate such that the osteointegrative portion is attached to the body portion upon fabrication of the object. In the process, digitized fillets directly attached to digitized struts of the digitized porous geometries at interfaces between the digitized osteointegrative volume and the digitized substrate may be created via the one or more computer processors. The digitized struts may correspond to struts of the porous geometries of the object, and the digitized fillets may correspond to fillets of the object.

In some arrangements according to any of the foregoing, in the process, radius settings for the digitized fillets may be set based on determined stress concentration factors at the respective interfaces between the digitized osteointegrative volume and the digitized substrate.

In some arrangements according to any of the foregoing, the digitized struts may be randomly distributed and oriented.

In some arrangements according to any of the foregoing, the created digitized fillets may be confined to areas within a predetermined, e.g., preset, distance of the digitized substrate that is less than a maximum thickness of the digitized osteointegrative volume measured normal to a digitized surface of the digitized substrate on which the digitized osteointegrative volume is located. The corresponding elements of the object, e.g., medical implant, fabricated based on the design would have the same relative locations to each other as their digitized counterparts.

In some arrangements according to any of the foregoing, the created digitized fillets may be confined to a digitized interface layer within the digitized osteointegrative volume that has a thickness in proportion to a thickness of the digitized osteointegrative volume measured normal to a digitized surface of the digitized substrate on which the digitized osteointegrative volume is located that varies across an area of the digitized surface.

In some arrangements according to any of the foregoing, any one or any combination of the digitized fillets may have a radius setting corresponding to a radius value for the physical radii of the interface region of the object approximately equal to 0.025 mm, 0.05 mm, 0.075 mm, 0.10 mm, 0.25 mm, or 0.50 mm.

In some arrangements according to any of the foregoing, any one or any combination of the digitized fillets may have a radius setting corresponding to a radius value for the physical radii of the interface region of the object of 0.5 mm or less.

In some arrangements according to any of the foregoing, any one or any combination of the digitized fillets may have a radius setting corresponding to a radius value for the physical radii of the interface region of the object in a range from 0.025 mm to 0.05 mm.

In some arrangements according to any of the foregoing, the creating of the digitized fillets may leave portions of a digitized surface of the digitized substrate extending between at least some of the digitized fillets free of contact with the digitized fillets and any portion of the digitized osteointegrative volume.

In some arrangements according to any of the foregoing, the corresponding elements of the object or other objected fabricated based on the created design would have the same relative locations to each other as their digitized counterparts.

In some arrangements according to any of the foregoing, an output file may be configured for use by an additive manufacturing machine for fabricating the object, e.g., medical implant. In this process, the output file may be stored, via the one or more computer processors. The output file may include digitized substrate data corresponding to the digitized substrate, digitized osteointegrative volume data corresponding to the digitized osteointegrative volume, and digitized fillet data corresponding to the digitized fillets.

BRIEF DESCRIPTION OF THE DRAWINGS

FIG. 1A is a cross-sectional view of a portion of an object having a regular porous portion as known in the art.

FIG. 1B is a cross-sectional view of a portion of another example of an implant having a randomized porous portion as known in the art.

FIG. 2A illustrates a cross-section of a portion of an object having a porous portion as known in the art.

FIG. 2B illustrates a cross-section of a configuration of a portion of an object having a porous portion in accordance with another aspect.

FIG. 3 is a schematic cross-sectional view of a modified configuration of a portion of an implant having a porous portion in accordance with an aspect.

FIGS. 4A-4C are cross-sectional elevation views of an object having a porous portion in accordance with other aspects.

FIG. 5A is a perspective view of a three-dimensional rendering of a section of an object having an interface section in accordance with another aspect.

FIG. 5B is a perspective view of a three-dimensional rendering of a section of an object having an interface section in accordance with another aspect.

FIG. 6 is a flow chart of a process for producing a model of an object having a porous portion in accordance with another aspect.

DETAILED DESCRIPTION

As used herein, the term "approximately" means that the value that the term modifies encompasses values within +/- 5% of the given value.

The present disclosure includes reference to processes carried out on a computer, with the illustrated example presenting steps carried out within a building application such as computer modeling or computer aided design ("CAD") software, e.g., NX, Solidworks, nTopology, or equivalents, in which a user may interface with the building application as described, e.g., in U.S. Patent No. 10,596,660, the disclosure of which is hereby incorporated herein by reference. However, any or all of the steps presented may optionally be carried out without any graphical presentation, particularly where such steps are carried out automatically by a computer. Any step or process in this disclosure or any combination thereof may, unless specified otherwise, be carried out by a computer carrying suitable CAD software at the direction of a human user, by a computing device having a processor and a non-transitory, computer readable medium carrying instructions that, when read by the processor, cause the processor to execute such steps or processes automatically, or by such a computing device in cooperation with a human user.

The computer-based design processes described herein can be used to create one or more manufacturing models that may be fabricated as physical products. Any fabrication method, including additive manufacturing, subtractive manufacturing, or a combination thereof, may be used as suitable for the size and shape of the output, meaning the final desired fabricated component, and the intended material or materials. An example includes using an additive manufacturing process as at least a first part of the fabrication process. In some arrangements, the additive manufacturing process may be, e.g., electron beam melting ("EBM"), selective laser sintering ("SLS"), selective laser melting ("SLM"), binder jetting, or blown powder fusion for use with metal powders.

When additive manufacturing by a powder-based fusion (PBF) process such as EBM, SLM, or SLS, a first layer of metal powder is deposited onto a substrate and then scanned with a high energy beam so as to sinter or melt the powder and create a portion of one or more predetermined physical porous geometries. Successive layers of the metal powder are then deposited onto previous layers of the metal powder and also respectively scanned with the high energy beam prior to the deposition of subsequent layers of the metal powder. The scanning and depositing of successive layers of the metal powder continues the building process of the predetermined physical porous geometries. Such continuation of the building process refers not only to a continuation of a predetermined physical porous geometry from a previous layer but also a beginning of a new predetermined physical porous geometry as well as or instead of the completion of a predetermined physical porous geometry, depending on the desired characteristics of the structure or structures to be fabricated.

The structures formed using this process may be partially porous and, if desired, have interconnecting pores to provide an interconnecting porosity. In some arrangements, the physical porous geometries may be defined by physical struts connected at vertices corresponding to digitized nodes within a CAD or other modeling program. The metal powder and thus the additively printed porous portion or portions preferably may be made of any one or any combination of cobalt chrome alloy, titanium or alloy, stainless steel, niobium, and tantalum. Thus, a mixture of desired mixed materials may be employed.

The high energy beam preferably may be an electron beam (e-beam) or laser beam and may be applied continuously to the powder or pulsed at a predetermined frequency. In some arrangements, the use of a laser or e-beam melting process may preclude the requirement for subsequent heat treatment of the structure fabricated by the additive manufacturing process, thereby preserving the initial mechanical properties of the additively manufactured porous portion. The high energy beam is emitted from a beam-generating apparatus to heat the metal powder sufficiently to sinter or at least partially melt the metal powder. High energy beam generation equipment for manufacturing such structures may be one of many currently available including the "Concept laser M2 Cusing" machines, GE Concept M2 Cusing Gen 2 machines, GE Arcam Q10 machines, 200W M2 Cusing (series 3), kW M2 Cusing (Series 3), Dual kW M2 Cusing (Series 5) MCP REALIZER, the EOS M270, TRUMPF TRUMAFORM 250, the ARCAM EBM S12 and Q10 machine, and the like. The beam generation equipment may also be a custom-produced laboratory device.

The porosity, pore density, pore size, and pore size distribution may be controlled from one location to another. It is important to note that successive powder layers may differ in the pores or portions of pores formed within such layers by varying factors used for high energy beam scanning of powder layers. Additionally, the porosity within a set of successive layers of powder may vary depending on the specific type of unit cell used within such successive layers of powder or by manipulating various dimensions of a given unit cell. In some arrangements, the porosity may be a gradient porosity throughout at least a portion of the fabricated structure. The beam generation equipment may be programmed to proceed in a random generated manner to produce an irregular porous construct but with a defined level of porosity. Pseudo-random geometries may be formed by applying a perturbation to the vertices of digitized porous geometries when preparing model build structures corresponding to the 3D structure to be fabricated. In this manner, the shapes and sizes of the physical porous geometries may be randomized.

In some arrangements, additively manufactured porous structures may be in the form of overlapping lines of solidified powder as disclosed in U.S. Patent No. 7,537,664, the disclosure of which is hereby incorporated by reference herein. In some arrangements, additively manufactured porous structures may be in the form of cellular structures defined by repeating formed porous geometries corresponding to digitized unit cells as disclosed in U.S. Patent Nos. 10,525,688 and 9,180,010, the disclosures of which are hereby incorporated by reference herein. In some arrangements, additively manufactured porous structures may be in the form of a mesh or chainmail as disclosed in U.S. Patent No. 10,596,660 and U.S. Patent No. 10,888,362, the disclosure of which is hereby incorporated by reference herein as if fully set forth herein. In some arrangements, additively manufactured structures may be formed with or even on flanges in the manner as disclosed in U.S. Patent No. 10,456,262, the disclosure of which is hereby incorporated by reference herein.

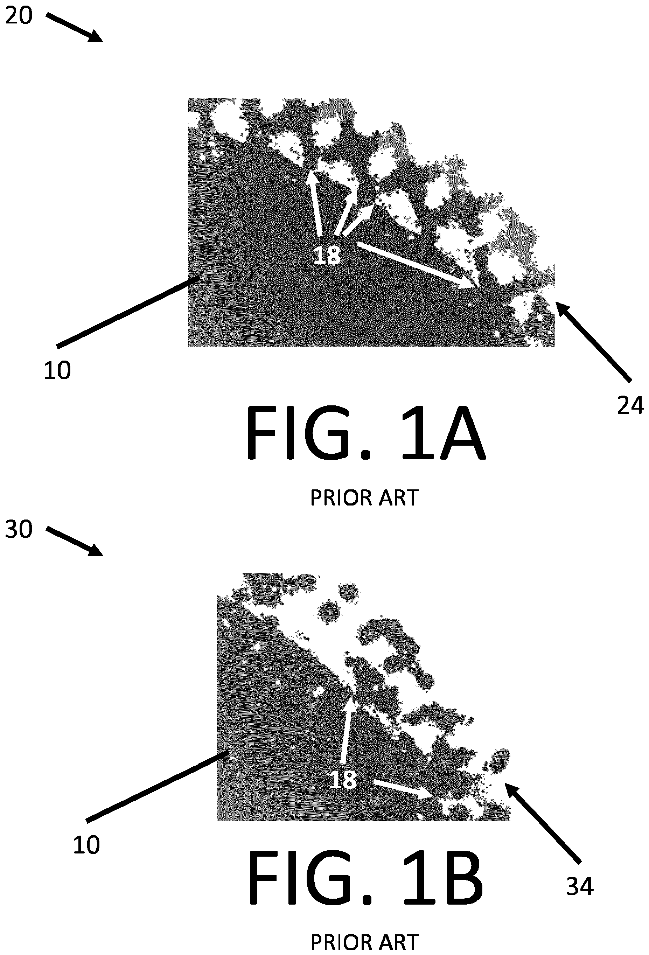

Referring now to the figures, as shown in FIG. 1A, implant section 20 is an example of a porous structure known in the art having an undesirable interface between porous and solid portions forming the porous structure. Implant 20 has or at least is intended to have regular cellular structure 24 of linear struts providing a porous, osteointegrative portion on top of solid substrate 10, which is a portion of a solid structure or body of the implant. As shown in FIG. 1B, implant section 30 is another example of a porous structure known in the art in which this implant section includes substrate 10 and irregular porous portion 34 attached to the substrate. Irregular porous portion 34 of implant section 30 is formed by offsetting the locations of digitized vertices of a digitized regular cellular structure such that the irregular porous portion includes struts placed in an irregular or even random distribution of locations and orientations extending from substrate 10. In both implant configurations, porous portions 24, 34 include notches 18 where struts or other solid elements that collectively provide the pores of the porous portions 24, 34 intersect each other or meet with substrate 10. Porous portions 24, 34 may be modified according to processes described below to reduce or eliminate stress concentrations likely to result in notches 18. The images used to illustrate the following processes variously depict regular and irregular structures, but the following processes may be applied in generally the same manner to optimize regular porous structures like porous portion 24 and irregular porous structures like porous portion 34. Moreover, the following processes may be used to modify TRITANIUM® porous surfaces by Stryker Corporation and other similar osteointegrative structures.

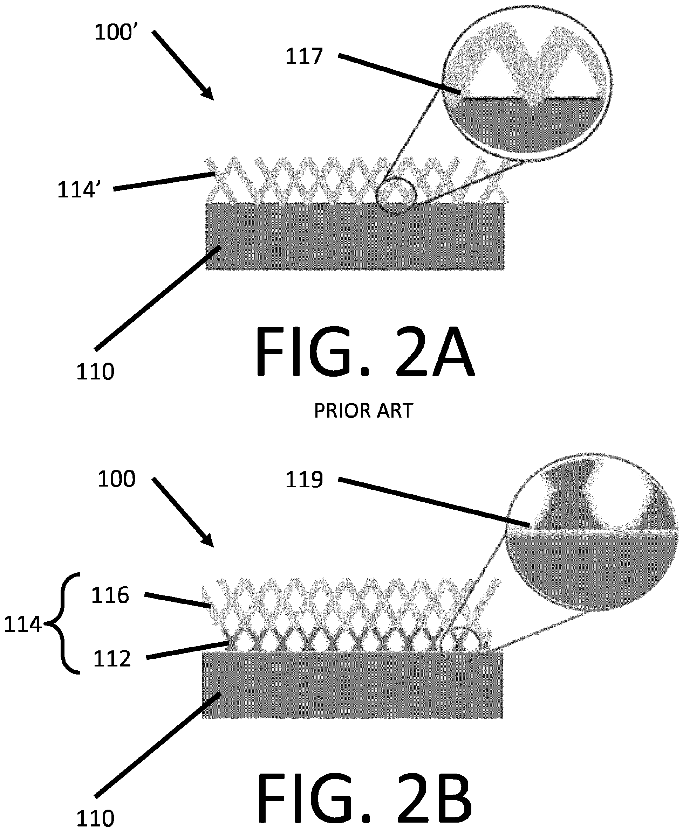

Unmodified surface configuration 100' as shown in FIG. 2A is a representation of a computer model of a portion of an object, which may be a medical implant, configured for additive manufacturing, and is a precursor of modified surface configuration 100 described further herein. In some arrangements when the object is a medical implant, the medical implant may be in the form of a shoulder implant, a hip stem component, a patellofemoral component, a tibial component, a spinal implant, a knee implant, a trauma plate, a foot implant, an ankle implant, or an acetabular cup. Unmodified surface configuration 100' may be generated according to any known method for designing and generating computer models of implants or other objects for additive manufacturing or by any yet to be developed method that is otherwise able to be modified as described herein. Unmodified surface configuration 100' includes digitized substrate 110 and digitized porous portion 114', which may be a digitized osteointegrative structure of a digitized medical implant, attached to the digitized substrate. Digitized porous portion 114' includes areas of high stress concentration 117 at digitized corners formed where the digitized struts contact digitized substrate 110 as shown in the illustrated example. Such areas of high stress concentration 117 are narrow corners between digitized porous portion 114' and digitized substrate 110 where notches 18 are relatively likely to occur in a physical object fabricated according to the design of unmodified surface configuration 100'. Digitized substrate 110 is generally consistent with digitized substrate 10 as described above, and digitized porous portion 116 may be generally consistent with above-described regular cellular structure 24 or irregular porous portion 34 and may be a digitized osteointegrative structure.

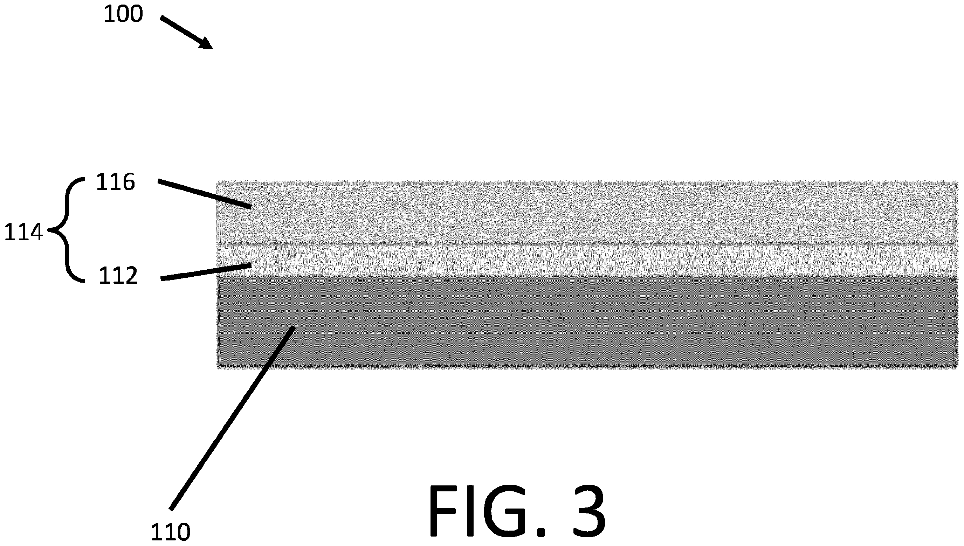

Turning to FIGS. 2B and 3, modified surface configuration 100 is a representation of a computer model of a portion of an object, which may be a medical implant, configured for fabrication by additive manufacturing. The computer model of modified surface configuration 100 includes digitized substrate 110, digitized non-interface porous layer 116, and digitized interface portion 112 between the digitized substrate and the digitized porous portion, in which the digitized substrate is a solid structure or body of an implant. Digitized interface portion 112 and digitized non-interface porous layer 116 together provide a modified digitized porous portion 114, which may be an osteointegrative portion and which may extend a same distance from digitized substrate 110 as unmodified digitized porous portion 114' of unmodified surface configuration 100'. Digitized interface portion 112 is a portion in which preexisting digitized porous geometries have been modified to mitigate stress concentrations according to processes explained below.

Regions or an entirety of digitized porous portion 114' adjoining digitized substrate 110 may be analyzed, e.g., through finite element analysis as described further herein, to identify some or all notches 118 therein. Unmodified surface configuration 100' may be converted to modified surface configuration 100 by adding digitized fillets 119 at the areas of high stress concentration 117 to convert the analyzed part of digitized porous portion 114' into digitized interface portion 112, leaving the remainder of the digitized porous portion as digitized non-interface porous layer 116 as shown in FIG. 2B.

In implants manufactured according to modified surface configuration 100 shown in FIG. 2B, digitized non-interface porous layer 116 maintains a porosity and geometry designed to promote osteointegration. The addition of digitized fillets 119 in digitized interface layer or interface portion 112 reduces the porosity of digitized interface portion 112 slightly below the porosity of digitized non-interface porous layer 116 but increases overall strength and durability of digitized interface portion 112 by mitigating stress concentrations therein. The addition of digitized fillets 119 therefore significantly reduces the risk of modified digitized porous portion 114 fracturing or separating from digitized substrate 110 while mostly or entirely maintaining the overall porous configuration present in digitized precursor porous portion 114', and in the example of a medical implant, the osteointegrative facility of digitized porous portion 114.

Digitized fillets 119 may be set with any radius setting appropriate for the scale of the part being modified. In the examples illustrated and described herein, which generally concern the porous interface portions of orthopedic implants, fillet radii of equal to or about 0.025 mm, 0.050 mm, 0.075 mm, 0.100 mm, 0.250 mm, or 0.500 mm are contemplated. For the same examples, fillet radii in the ranges of 0.500 mm or less, 0.050 mm or less, from 0.025 mm to 0.500 mm, and from 0.025 mm to 0.050 mm are contemplated.

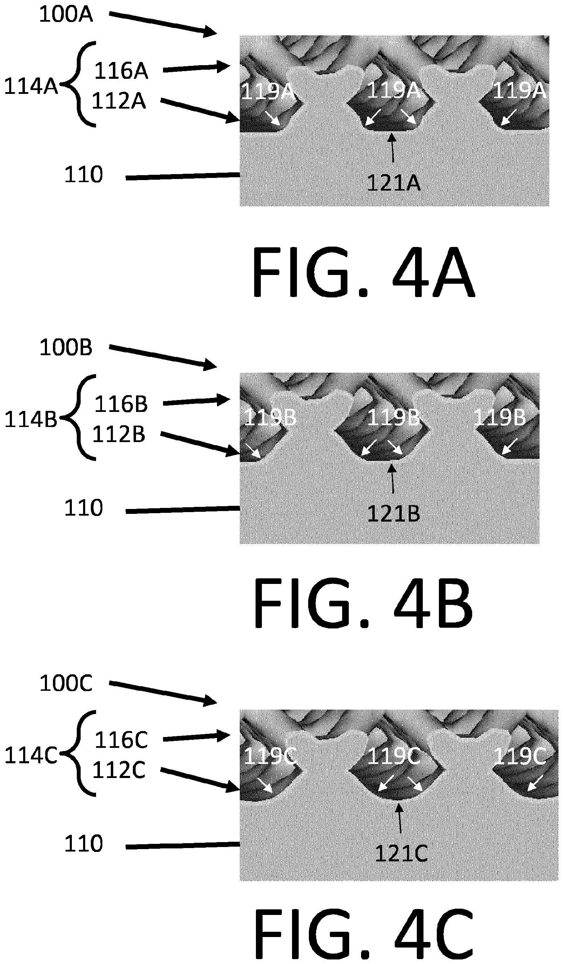

FIGS. 4A-4C illustrate modified surface configurations 100A, 100B, 100C having fillets 119A, 119B, 119C of varying radii. Each surface configuration 100A, 100B, 100C is modeled upon substrate 110 and includes a respective modified digitized porous portion 114A, 114B, 114C that further includes a digitized interface layer or interface portion 112A, 112B, 112C and a digitized non-interface porous layer 116A, 116B, 116C. Modified surface configurations 100A, 100B, 100C are thus examples of possible implementations of modified surface configuration 100 described above.

Modified surface configurations 100A, 100B, 100C are all derived from an identical unmodified surface configuration 100' and are therefore identical to one another except for the radii of fillets 119A, 119B, 119C. Fillets 119B illustrated in FIG. 4B have larger radii than fillets 119A of FIG. 4A, but smaller radii than fillets 119C of FIG. 4C. Fillets 119A, 119B of FIGS. 4A and 4B are small enough relative to the spacing between the struts to leave a flat surface area 121A, 121B on top of the substrate between the fillets 119A, 119B. By contrast, fillets 119C of FIG. 4C are large enough to create interference areas 121C where spaced apart fillets 119C overlap one another. It has been observed that for geometries resembling those depicted in FIGS. 4A-4C, modified surface configuration 100B of FIG. 4B results in greater reduction in stress concentrations and better overall part strength than modified surface configurations 100A and 100C of FIGS. 4A and 4C. More generally, stress concentrations in modified surface configurations 100 generally decrease as radii of fillets 119 increase until the radii become so large that fillets 119 added to struts that contact substrate 110 relatively far from one another begin to overlap, at which point increasing the radii further will result in less reduction of stress concentrations. As such, for some arrangements of modified surface configurations 100, stress concentrations can be minimized by adding fillets 119 with radii small enough to leave portions of a surface of substrate 110 contacted by modified digitized interface portion 114 free of fillets 119 or contact by struts between at least some of the fillets 119. Stated another way, the surface of substrate 110 to which digitized interface layer 112 joins digitized porous portion 114 includes portions that are located between fillets 119 and free of contact by any portion of digitized interface layer 112.

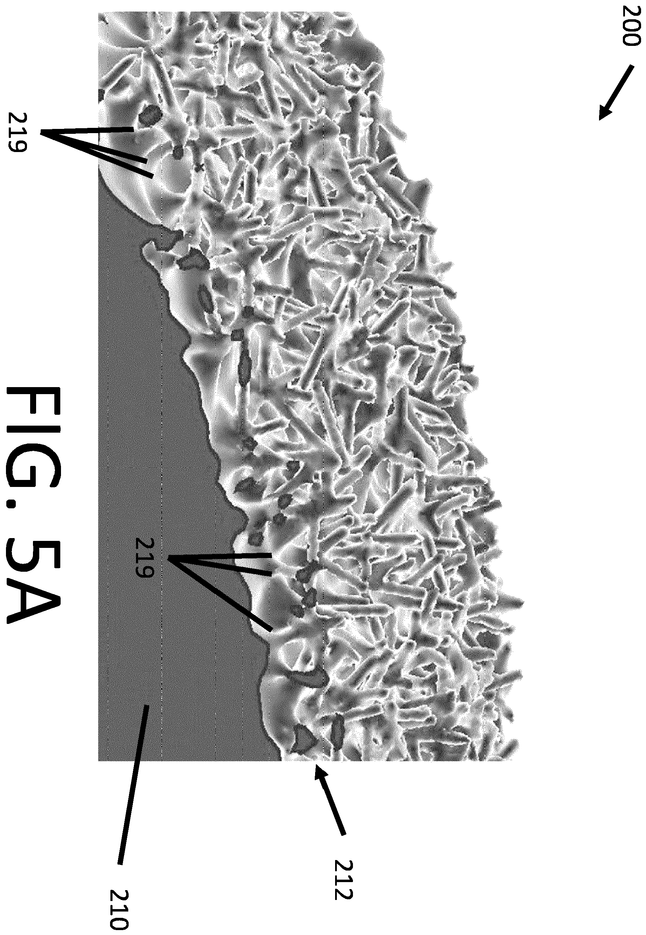

An example of a computer rendering of a perspective view of a section of modified surface configuration 200 is illustrated by FIG. 5A. Modified surface configuration 200 is generally alike to modified surface configuration 100, with like numerals referring to like elements (i.e., digitized substrate 210 is generally alike to digitized substrate 110), except that modified surface configuration 200 features an irregular arrangement of struts whereas modified surface configuration 100 has a regular arrangement of struts. As shown, digitized interface portion 212 is directly atop digitized substrate 210. Multiple rounded digitized fillets 219 can be observed in digitized interface portion 212. The relative proportions of digitized interface portion 212 to a digitized porous portion that may be built thereon can be varied from what is shown in the illustrated examples according to factors including the desired mechanical strength and durability of the implant, the desired overall porosity and osteointegrative facility of a modified osteointegrative layer, the radius setting or settings selected for digitized fillets 219, the geometry selected for forming the porous layer, and any manufacturing considerations. The relative proportions of digitized interface portion 112 to modified digitized porous portion 114 can be varied in the same way and with respect to the same considerations. In some examples, digitized interface portion 212 may extend throughout an entirety of modified digitized porous portion 114 such that no distinct porous portion remains within the modified digitized porous portion. In various other examples, digitized interface portion 212 may be of a predetermined, uniform thickness as measured by distance digitized from substrate 210, digitized interface portion 212 may be a uniform proportion of modified digitized porous portion throughout a part or an entire extent of the modified digitized porous portion, or digitized interface portion 212 may vary in either one or both of absolute thickness and proportion of the digitized modified porous portion between locations as necessary to achieve desired mechanical or, in the case of a medical implant, osteointegrative properties of the modified osteointegrative portion at various locations.

The example shown in FIG. 2B shows modified digitized porous portion 114 resulting from modification of unmodified digitized surface configuration 100' in which unmodified digitized porous portion 114' is provided by a regular, repeating cellular structure defined by linear struts extending from substrate 110. In contrast, the example shown in FIG. 5A shows a modified digitized porous portion resulting from modification of an unmodified surface configuration that includes an unmodified porous portion provided by an irregular mass of randomly distributed and randomly oriented struts extending from digitized substrate 210.

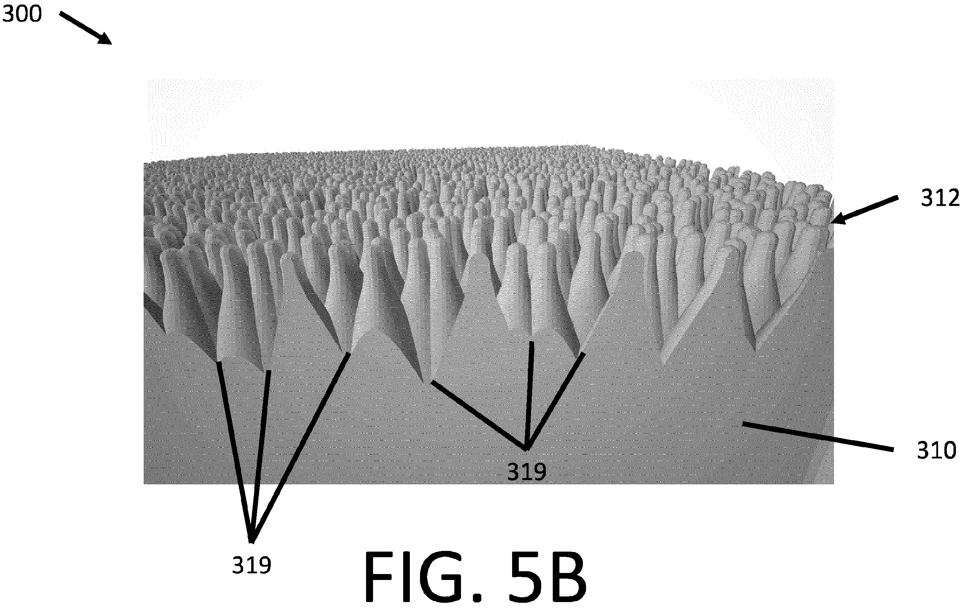

FIG. 5B in turn shows another modified surface configuration 300, which is generally alike to modified surface configurations 100, 200, with like numerals corresponding to like figures. Modified surface configuration 300 thus likewise includes modified digitized interface portion 312 upon digitized substrate 310, with digitized fillets 319 added at locations where a precursor, unmodified version of a digitized interface portion met digitized substrate 310 at concavities that likely would have resulted in stress concentrations in a corresponding, additively manufactured product. Unlike modified surface configuration 200, modified surface configuration 300 includes modified digitized interface portion 312 that is defined by a blending or filleting of the digitized strut to the surface of digitized substrate 310, rather than the development of a butt joint between each of the digitized struts and digitized substrate. The degree of blending of the digitized struts of the modified digitized interface portion 312 is a function of the angle of protrusion of the digitized strut relative to digitized substrate 310 and a blend radius setting corresponding to a radius value of a physical fillet to be prepared based on the modified digitized interface portion.

FIGS. 5A and 5B are each cut off at the outer edge of the respective modified digitized interface portion 212, 312 depicted therein. However, modified surface configurations 200, 300 both include a non-interface digitized porous layer on an opposite side of their respective modified digitized interface portions 212, 312 from their respective digitized substrates 210, 310. The non-interface digitized porous layers of modified surface configurations 200, 300 are generally alike to non-interface digitized porous layer 116 of modified surface configuration 100, except for the arrangement of the struts and the shapes of fillets therein. In this manner, the non-interface digitized porous layer of surface configuration 200 together with modified digitized interface portion 212 provides modified digitized porous portion of surface configuration 200. Similarly, the non-interface digitized porous layer of surface configuration 300 together with modified digitized interface portion 312 provides a modified digitized porous portion of surface configuration 300. The modified digitized porous portions of surface configurations 200, 300 are generally alike to the modified digitized porous portion 114 of surface configuration 100, except for the configurations of the struts therein. Thus, surface configurations 100, 200, 300, are generally similar to each other and may be created by generally the same processes, except that each surface configuration 100, 200, 300 results from application of those processes to input or precursor files or models having different arrangements of struts.

The modification processes described herein are equally applicable to unmodified porous portions, e.g., osteointegrative portions, provided by regular or irregular arrangements of linear struts. Except where specifically stated otherwise, the modification processes described herein are also applicable to unmodified porous portions, e.g., osteointegrative portions, provided by any other geometries, including regular or irregular arrangements of non-linear struts, porous bodies provided by arrangements of elements other than struts, or porous bodies provided by geometries that are not formed by arrangements of discrete, repeating elements.

Analysis of digitized porous portion 114', or the corresponding precursor to modified surface configurations 200, 300, to find areas of high stress concentration 117 may include finding stress concentrations within the digitized unmodified porous portion 114', or the corresponding precursor to modified surface configurations 200, 300, that are consistent with areas of high stress concentration 117. Calculating the stress concentration factor (Kt) of a given geometry can be accomplished through the application of finite element analysis software to find relatively high stress concentrations, e.g., based on maximum stress calculations for some or all regions at the interface of substrate 110, 210, 310 and digitized non-interface porous layer 116, or the corresponding porous portions for modified surface configurations 210, 310, using Equation 1. Relatively high stress concentrations are consistent with the presence of notches. Alternatively, likely locations of areas of high stress concentration 117 may be identified by algorithmic analysis of geometry of digitized porous portion 114', or the corresponding porous portions for modified surface configurations 210, 310, such as by finding any locations at which two identifiable surfaces, optionally each being of a predetermined minimum size, intersect at an angle narrower than a predetermined minimum angle or intersect without a transition of at least a minimum radius value. Such processes are only examples, and likely locations of stress concentrations may be found by any available form of analysis known to those skilled in the art. Digitized fillets 119, 219, 319 can be added where notches are likely to occur in order to reduce stress concentrations, such as by use of topology optimization software or algorithms.

Digitized fillets 119, 219, 319 all may be prepared using a set radius value or varying set radius values. For example, radii of individual digitized fillets 119, 219, 319 may be selected as necessary to reach a target maximum stress concentration at the location where each digitized fillet 119, 219, 319 will be placed or according to other mathematical or algorithmic analyses of the geometry surrounding each digitized fillet's 119, 219, 319 intended location.

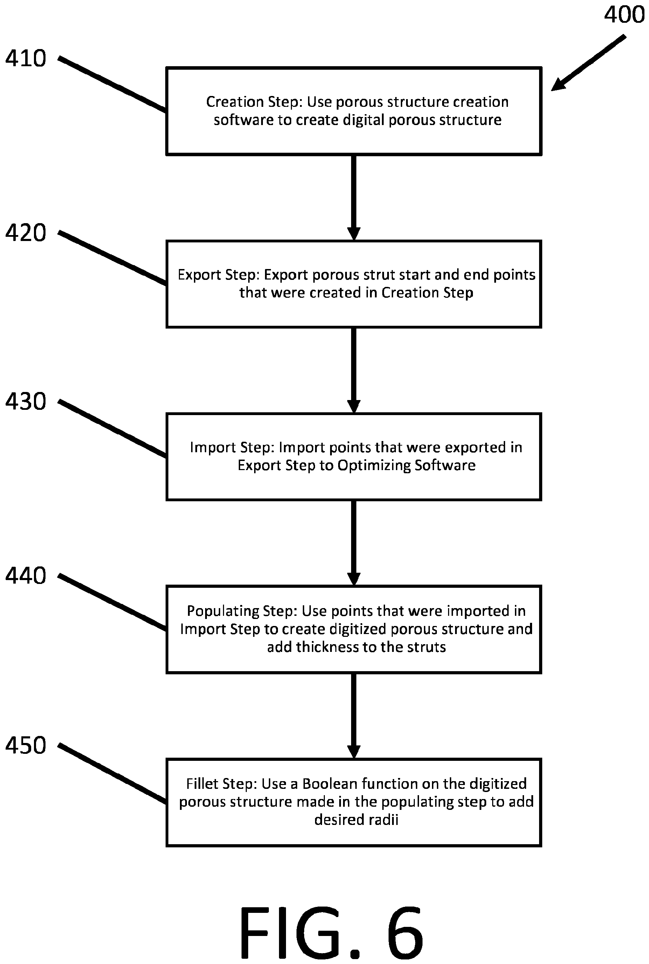

FIG. 6 shows an example process 400 for creating modified surface configuration 100, 200, 300 from struts. At creation step 410, data are created from which a computer model of a digitized porous portion, such as digitized porous portion 114' or the digitized porous portion used to prepare modified surface configurations 200, 300, may be derived. Creation step 410 may be according to any known processes for populating struts throughout a computer model to create a porous layer on top of a substrate portion of a solid body of an implant model. At export step 420, the data created in creation step 410 may be exported from the creation software and, at import step 430, imported into optimizing software. However, export step 420 and input step 430 are optional because in some examples, the creation software and optimizing software may be the same program.

In examples wherein the digitized porous portion is provided by regular or irregular arrangements of struts, the data exported in export step 420 and imported in import step 430 may be start points and end points of digitized struts, e.g., the digitized struts attached to digitized substrate 110, 210, 310. When the data is start points and end points of digitized struts, the optimizing software creates a computer model of a regular or irregular cellular structure formed by the struts by populating the planned three-dimensional geometries of the struts according to the start points and the end points in populating step 440. Populating step 440 may be unnecessary if the optimizing software receives a complete computer model of the porous portion.

Modification process 400 ends with fillet step 450. Fillet step 450 is a step of identifying areas of relatively high stress concentration where struts meet the digitized substrate 110, 210, 310, e.g., based on maximum stress calculations for some or all regions at the interface of substrate 110, 210, 310 and the digitized porous portion 114, or the corresponding layers of modified surface configurations 200, 300, using Equation 1. Fillet step 450 further includes adding digitized fillets 119, 219, 319 at the identified areas of relatively high stress concentration in a manner similar to what was described above with regard to conversion of digitized porous portion 114' to modified digitized porous portion 114. Fillet step 450 can include similar or the same analyses regardless of the type of porous structure upon which it is executed.

Although the concepts herein have been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

**Claims**

1. A method of fabrication of a medical implant comprising the steps of:
   producing a porous structure, a solid structure, and an interface region directly attached to each of the porous structure and the solid structure using a stored output file configured for providing instructions to an additive manufacturing machine for fabricating the medical implant, the porous structure, the solid structure, and the interface region forming at least part of the medical implant, the output file being prepared by the steps of:

   forming, by one or more computer processors, a plurality of digitized struts corresponding to formed struts of the porous structure of the medical implant and defining a porous CAD volume;
   forming, by the one or more computer processors, a solid model region corresponding to the solid structure of the medical implant and defining a solid CAD volume;
   forming, by the one or more computer processors, digitized radii corresponding to physical radii of the interface region of the medical implant to be formed and defining an interface volume, the digitized radii being directly attached to the plurality of digitized struts and to the solid model region;
   generating, by the one or more computer processors, a computer-generated model of the medical implant configured for additive manufacturing, the computer-generated model including data corresponding to the digitized struts, the solid model region, and the digitized radii; and
   storing, by the one or more computer processors, the computer-generated model of the medical implant into the output file.

2. The method of claim 1, wherein the output file is further prepared by the step of identifying concave areas at which digitized struts meet the solid model region.

3. The method of claim 1 or claim 2, wherein the entirety of the interface volume is contiguous with the solid CAD volume.

4. The method of claim 3, wherein the interface volume is defined within a predetermined distance from the solid CAD volume.

5. The method of claim 4, wherein the interface volume has a thickness that is of a predetermined proportion to a thickness of the porous CAD volume as measured normal to a surface of the solid CAD volume with which the interface volume is contiguous across an area of the surface of the solid CAD volume.

6. The method of any one of claims 1-5, wherein the step of identifying the concave areas includes conducting finite element analysis of at least part of the porous CAD volume to find stress concentrations having characteristics consistent with concave notches in the fabricated medical implant.

7. The method of any one of claims 1-6, wherein the step of forming the digitized radii includes setting all of the digitized radii to a same radius setting corresponding to a radius value for the physical radii of the interface region of the medical implant.

8. The method of any one of claims 1-6, wherein the step of forming the digitized radii includes placing, by the one or more computer processors, each of the digitized radii at respective locations within the interface volume having a determined initial stress concentration factor above a preset minimum threshold and assigning, by the one or more computer processors, each of the respective digitized radii a respective radius setting that reduces the determined initial stress concentration factor at the respective locations to a determined new stress concentration factor that is less than or equal to a preset maximum threshold.

9. The method of claim 8, wherein the preset minimum threshold equals the preset maximum threshold, and wherein each of the digitized radii at respective locations within the interface volume having a determined initial stress concentration factor above the preset minimum threshold is assigned a respective radius setting that reduces the determined new stress concentration factor to less than the preset maximum threshold.

10. The method of any one of claims 1-6, wherein the step of forming the digitized radii includes setting radii settings for the digitized radii based on multiple factors, at least two of the multiple factors being selected from the group consisting of respective angles between the digitized struts onto which the digitized radii are being formed and the solid model region, the respective thicknesses of the digitized struts, the material of the porous structure of the medical implant to be fabricated, the material of the solid structure of the medical implant to be fabricated, and the material of the physical radii to be fabricated.

11. The method of any one of claims 1-10, wherein any one or any combination of the digitized radii are set to a radius setting corresponding to a radius value for the physical radii of the interface region of the medical implant approximately equal to 0.025 mm, 0.05 mm, 0.075 mm, 0.10 mm, 0.25 mm, or 0.50 mm.

12. The method of any one of claims 1-10, wherein any one or any combination of the digitized radii are set to a radius setting corresponding to a radius value for the physical radii of the interface region of the medical implant of 0.5 mm or less.

13. The method of claim 12, wherein any one or any combination of the digitized radii are set to a radius setting corresponding to a radius value for the physical radii of the interface region of the medical implant in a range from 0.025 mm to 0.05 mm.

14. The method of any one of claims 1-13, wherein the step of forming the digitized radii leaves portions of a digitized surface of the solid model region extending between at least some of the digitized radii free of contact with any of the digitized struts and with any of the digitized radii, and wherein the digitized surface of the solid model region corresponding to a surface of the solid structure.

15. A medical implant prepared using a stored output file configured for providing instructions to an additive manufacturing machine for fabricating the medical implant comprising:

a porous structure including a plurality of struts defining pores;
a solid structure; and
an interface region attaching the porous structure to the solid structure, the interface region including a plurality of radii, each of the radii being directly attached only to a respective strut of the plurality of struts of the porous structure and to the solid structure such that the porous structure and the solid structure are directly attached only to each other and by the plurality of radii, wherein the output file is prepared by the steps of:

forming, by one or more computer processors, a plurality of digitized struts corresponding to formed struts of the porous structure of the medical implant and defining a porous CAD volume (114);
forming, by the one or more computer processors, a solid model region (110, 210, 310) corresponding to the solid structure of the medical implant and defining a solid CAD volume;
forming, by the one or more computer processors, digitized radii (119, 219, 319) corresponding to physical radii of the interface region of the medical implant to be formed and defining an interface volume, the digitized radii being directly attached to the plurality of digitized struts and to the solid model region;
generating, by the one or more computer processors, a computer-generated model of the medical implant configured for additive manufacturing, the computer-generated model including data corresponding to the digitized struts, the solid model region, and the digitized radii; and
storing, by the one or more computer processors, the computer-generated model of the medical implant into the output file.

20

10

18

24

# FIG. 1A

PRIOR ART

30

10

18

34

# FIG. 1B

PRIOR ART

100'

117

114'

110

# FIG. 2A

PRIOR ART

100

119

116

114

112

110

# FIG. 2B

# FIG. 3

FIG. 4A

FIG. 4B

FIG. 4C

FIG. 5A

EP 4 275 657 A1

300

312

310

319

319

# FIG. 5B

400

410

Creation Step: Use porous structure creation
software to create digital porous structure

420

Export Step: Export porous strut start and end points
that were created in Creation Step

430

Import Step: Import points that were exported in
Export Step to Optimizing Software

440

Populating Step: Use points that were imported in
Import Step to create digitized porous structure and
add thickness to the struts

450

Fillet Step: Use a Boolean function on the digitized
porous structure made in the populating step to add
desired radii

# FIG. 6

Europäisches
Patentamt
European
Patent Office
Office européen
des brevets

# EUROPEAN SEARCH REPORT

Application Number

EP 23 17 3220

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/296350 A1 (HAMZEY RAMI [US] ET AL) 18 October 2018 (2018-10-18) | 1,3-5, 14,15 | INV. A61F2/30 |
| A | * paragraph [0050] – paragraph [0071] *<br>* paragraph [0079] – paragraph [0082] *<br>* figures 1-5 * | 2,6-13 | B33Y80/00 |
| A | US 2021/251764 A1 (MOORE COWAN H [US] ET AL) 19 August 2021 (2021-08-19)<br>* paragraph [0018] *<br>* paragraph [0080] – paragraph [0089] *<br>* figures 2A-2D * | 1,15 | |
| A | WO 2021/113797 A1 (FORMAE INC [US]) 10 June 2021 (2021-06-10)<br>* paragraph [0063] – paragraph [0071] *<br>* paragraph [0077] – paragraph [0086] *<br>* figures 4A,5A,9B * | 1,15 | |
| A | US 2018/333780 A1 (KLEIN ROBERT W [US] ET AL) 22 November 2018 (2018-11-22)<br>* paragraph [0062] – paragraph [0067] *<br>* figure 5A * | 1,15 | TECHNICAL FIELDS SEARCHED (IPC)<br><br>A61F<br>B33Y |

The present search report has been drawn up for all claims

| Place of search | Date of completion of the search | Examiner |
|---|---|---|
| Munich | 4 September 2023 | Storer, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

**EP 4 275 657 A1**

## ANNEX TO THE EUROPEAN SEARCH REPORT
## ON EUROPEAN PATENT APPLICATION NO.

EP 23 17 3220

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2023

| Patent document cited in search report | | Publication date | Patent family member(s) | | Publication date |
|---|---|---|---|---|---|
| US 2018296350 | A1 | 18-10-2018 | BR 112019008299 | A2 | 08-10-2019 |
| | | | CN 110418624 | A | 05-11-2019 |
| | | | CN 114366265 | A | 19-04-2022 |
| | | | EP 3531981 | A1 | 04-09-2019 |
| | | | JP 6960461 | B2 | 05-11-2021 |
| | | | JP 2019531866 | A | 07-11-2019 |
| | | | JP 2022000292 | A | 04-01-2022 |
| | | | US 2018296350 | A1 | 18-10-2018 |
| | | | US 2021307909 | A1 | 07-10-2021 |
| | | | WO 2018081073 | A1 | 03-05-2018 |
| US 2021251764 | A1 | 19-08-2021 | US 10070962 | B1 | 11-09-2018 |
| | | | US 10098746 | B1 | 16-10-2018 |
| | | | US 2019046322 | A1 | 14-02-2019 |
| | | | US 2021251764 | A1 | 19-08-2021 |
| WO 2021113797 | A1 | 10-06-2021 | CN 115003256 | A | 02-09-2022 |
| | | | EP 4069151 | A1 | 12-10-2022 |
| | | | US 2022409379 | A1 | 29-12-2022 |
| | | | WO 2021113797 | A1 | 10-06-2021 |
| US 2018333780 | A1 | 22-11-2018 | AU 2018203479 | A1 | 06-12-2018 |
| | | | EP 3403747 | A1 | 21-11-2018 |
| | | | US 2018333780 | A1 | 22-11-2018 |
| | | | US 2022226897 | A1 | 21-07-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63341092
- US 10596660 B
- US 7537664 B
- US 10525688 B
- US 9180010 B
- US 10888362 B
- US 10456262 B